# EUROPEAN PATENT APPLICATION

(11) **EP 2 008 514 A1**
(43) Date of publication of application: **31.12.2008**
(21) Application number: 08011453.1
(22) Date of filing: 24.06.2008
(51) Int. Cl.: A01K 67/027, C12N 15/00

(54) **Method of constructing nucleus-implanted egg, parthenogenetic embryo and parthenogenetic mammal**

(30) Priority: 29.06.2007 JP 2007171707
(71) Applicant: Tokyo University of Agriculture, Setagaya-ku Tokyo 156-0054 (JP)
(72) Inventor: Kono, Tomohiro, Tokyo 156-0054 (JP); Kawahara, Manabu, Tokyo 156-0054 (JP)
(74) Representative: Hiebl, Inge Elisabeth

(57) **Abstract**

There is provided a nucleus-implanted egg that is a nucleus-implanted egg having 2 haploid genome sets derived from mammal ova and shows excellent production efficiency of adults. There is also provided a method of constructing a nucleus-implanted egg having haploid genome sets derived from an ng ovum and an fg ovum, which comprises the steps of (1) introducing a non-grown stage egg (ng ovum) into a nucleus-deleted egg in a germinal vesicle stage (GV stage egg) and then developing them to an MII phase (second meiosis metaphase) by in vitro culture for development to prepare a first nucleus-implanted egg, and (2) extracting MII phase chromosomes from said first nucleus-implanted egg and introducing it into other MII phase egg (fg ovum) to prepare a second nucleus-implanted egg, wherein an ovum in which both (A) a region (region A) that controls expressions of H19 gene and Igf2 gene and (B) a region (region B) that controls expressions of Gtl2 gene and Dlk1 gene are missing is used as the ng ovum or fg ovum, and an adult production rate is significantly improved from that of a conventional method.

## Description

### BACKGROUND OF THE INVENTION

### (i) Field of the Invention

The present invention relates to a method of constructing a nucleus-implanted egg. More specifically, it relates to a method of constructing a nucleus-implanted egg produced from maternal genomes alone. The present invention also relates to a method of constructing a parthenogenetic embryo from a nucleus-implanted egg. Further, the present invention relates to a method of producing a parthenogenetic mammal from the above parthenogenetic embryo.

### (ii) Description of the Related Art

Mammals perform ontogeny by fertilization of ova and sperm, and the ontogeny is never completed by ova alone, which means that the genomes of sperm and eggs are vitally different in function. It is said that the above functional difference is due to the existence of groups of genes (imprinted genes) which are identical but exhibit entirely different expressions depending upon whether they are from sperm or they are from ovum as a result of chemical DNA modification imprinted posteriori during the generation of germ cells. In fact, oocytes of neonates have not undergone the above gene modification, and a number of genes exhibit gene expression patterns like those derived from a sperm.

For analyzing the expression patterns of imprinted genes, the we have proposed a method of constructing a nucleus-implanted ovum from a genome derived from an oocyte of a neonate of a mouse and a genome of an ovum derived from a maturated female mouse (see "Genomic Imprinting during Oogenesis and Embryonic Development" by Tomohiro Kono, "Proteins, Nucleic Acid and Enzymes" Vol. 43, No. 4 (1998), pp. 267 to 274.)

The above method comprises the steps of (1) introducing neonate oocyte (ng ovum) into a nucleus-deleted egg at a germinal vesicle stage (GV stage) and then developing the oocyte to an MII phase (second meiosis metaphase) by in vitro culture for maturation to prepare a first nucleus-implanted egg, and (2) extracting an MII phase chromosome from the above first nucleus-implanted egg and introducing it into other MII phase egg (fg ovum) to prepare a second nucleus-implanted egg. The second nucleus-implanted egg obtained by this method has a haploid genome set derived from the ng ovum and a haploid genome set derived from the fg ovum.

Neonate oocytes (ng ova) do not resume any meiosis by nature until they reach the last stage (mouse ova having a diameter of 60 µm) of ovum growth process, and their cell cycles are at a stop at a diplotene stage in the beginning phase of the first meiosis. We have found that when the above neonate oocyte (ng ovum) whose cell cycle is at a stop is introduced into the cytoplasm of a fully grown oocyte, it resumes meiosis, and the above method has been accordingly proposed.

The introduced gene derived from the ng ovum has not undergone chemical DNA modification imprinted posteriori during the ovum growth period, and it is expected that the second nucleus-implanted egg will be a useful material for analyzing the expression control of an imprinted gene. It has been confirmed that parthenogenetic embryo from the above second nucleus-implanted egg develops to a fetus at day 13.5 of gestation, which fetus morphologically normal comparison with a fetus derived from a fertilized egg. However, its further growth could not been confirmed.

By subsequent studies, the we have found that a nucleus-implanted egg having an ability to grow up to adulthood is obtained by using an ovum from which an imprinted gene that undergoes posteriori gene modification during sperm generation, particularly an H19 gene, has been deleted, as the ng ovum or fg ovum in the above method, filed a patent application regarding the finding (International Patent Publication WO2005/011371) and published it in a scientific document (Tomohiro Kono et al, Nature Vol. 428, No. 6985, pp. 860 to 864, 22 April 2004.)

Although this method is an excellent method in that a nucleus-implanted egg having an ability to grow up to adulthood is obtained, the proportion of produced adults to parthenogenetic embryos is only about 1.5%, and an improvement in production efficiency of adults have been desired.

An object of the present invention is to provide a nucleus-implanted egg that is a nucleus-implanted egg having 2 (two) haploid genome sets derived from mammal ova and shows excellent production efficiency of adults. Another object of the present invention is to provide a method of constructing a parthenogenetic embryo from the nucleus-implanted egg and a method of producing a parthenogenetic mammal from the parthenogenetic embryo.

The fg ovum or ng ovum used in the method described in W02005/011371 is close to a gene derived from a sperm in that an imprinted gene, particularly an H19 gene, is missing. However, it differs from the gene derived from a sperm, and production efficiency of adults from an obtained nucleus-implanted egg is low.

Thus, we have focused our attention on paternal expressions and maternal expressions of genes and made studies for bringing a genomic gene of the second nucleus-implanted egg having 2 haploid genome sets derived from ova close to a genomic gene in the fertilization of sperm and ovum.

As a result, it has been found that in the method described in the WO2005/011371, the production efficiency of adults can be improved significantly by use of an ovum in which both (A) a DNA methylation imprint region (region A) that controls expressions of H19 gene and Igf2 gene and (B) a DNA methylation imprint region (region B) that controls expressions of Gtl2 gene and Dlk1 gene are missing, as the ng ovum or fg ovum, and the present invention has been completed based on this new finding.

### SUMMARY OF THE INVENTION

That is, according to the present invention, there is provided a method of constructing a nucleus-implanted egg of a mammal, the nucleus-implanted egg having a haploid genome set derived from an ng ovum and a haploid genome set derived from an fg ovum, which comprises the steps of
(1) introducing a non-grown stage egg (ng ovum) into a nucleus-deleted egg in a germinal vesicle stage (GV stage egg) and then developing the obtained egg to an MII phase (second meiosis metaphase) by in vitro culture for development to prepare a first nucleus-implanted egg, and
(2) extracting all of MII phase chromosomes from said first nucleus-implanted egg and introducing it into other MII phase egg (fg ovum) to prepare a second nucleus-implanted egg,
wherein an ovum in which both (A) a DNA methylation imprint region (region A) that controls expressions of H19 gene and Igf2 gene and (B) a DNA methylation imprint region (region B) that controls expressions of Gtl2 gene and Dlk1 gene are missing is used as the ng ovum or fg ovum.

The present invention includes a method of constructing a parthenogenetic embryo, which comprises activating said second nucleus-implanted egg and then developing the same in vitro culture for development.

Further, the present invention includes a method of producing a parthenogenetic mammal, which comprises implanting and growing said parthenogenetic embryo in the uterus of a female mammal.

The reason why the production efficiency of adults can be improved in the present invention can be estimated as follows. That is, in mammals, identical genes or alleles are arranged in the same sequence on homologous chromosomes derived from paternal and maternal genes, genic expressions are equally exhibited from biparental alleles to take part in gene expressions of individuals.

However, some genes exhibit paternal expressions and some genes exhibit maternal expressions. For example, an embryo growth factor Igf2 (insulin like growth factor II) gene is expressed from a paternal locus (derived from a sperm) but is not expressed from a maternal locus (derived from an ovum). Meanwhile, an H19 gene located in a downstream to the Igf2 gene is expressed only from a maternal locus. Such a gene is called an imprinted gene. This is because the Igf2 gene and the H19 gene have in common an enhancer (gene expression enhancing sequence) located in a downstream to the H19 gene. This enhancer generally works dominantly over the H19 gene, and when an expression regulating region (region A) in an upstream of the H19 gene undergoes gene modification posteriori (DNA methylation) during the generation of sperm, it can no longer work on the H19 gene, and it comes to work on the Igf2 gene. As a result, there is established a relationship in which the expression of the H19 gene from a paternal gene is inhibited and the Igf2 gene is expressed from a paternal gene.

Similarly, a Dlk1 gene and a Gtl2 gene are imprinted genes expressed from a paternal locus and a maternal locus, respectively. An expression regulating region (region B) located in an upstream of the Gtl2 gene undergoes methylation during the generation of sperm, whereby expression regulation is established.

That is, in the general fertilization of sperm and ovum, the H19 and Gtl2 genes are expressed from a maternal gene and the Igf2 and Dlk1 genes are expressed from a paternal gene, whereby normal embryogenesis is performed, as shown in Table 1.

**Table 1**

| Gene | Igf2 | H19 | Dlk1 | Gtl2 |
|---|---|---|---|---|
| Sperm | O | × | O | × |
| Ovum | × | O | × | O |

| | | | | |
|---|---|---|---|---|
| O: Expression is promoted. ×: Expression is inhibited. | | | | |

In parthenogenesis between ova, it is expected that since the genes of both of the ova are derived from maternal genes, the H19 and Gtl2 genes expressed from a maternal gene are expressed excessively and the Igf2 and Dlk1 genes expressed from a paternal gene are not expressed, as shown in Table 2.

**Table 2**

| Gene | Igf2 | H19 | Dlk1 | Gtl2 |
|---|---|---|---|---|
| Ovum | × | O | × | O |
| Ovum | × | O | × | O |

| | | | | |
|---|---|---|---|---|
| O: Expression is promoted. ×: Expression is inhibited. | | | | |

Thus, it is expected that when an ovum in which both the region A and the region B are missing is used as the ng ovum or fg ovum, expression regulation close to general fertilization of sperm and ovum is performed and an adult production rate can be improved significantly, as shown in Table 3.

**Table 3**

| Gene | Igf2 | H19 | Dlk1 | Gtl2 |
|---|---|---|---|---|
| Ovum Having No Regions A and B | O | × | O | × |
| Ovum | × | O | × | O |

| | | | | |
|---|---|---|---|---|
| O: Expression is promoted. ×: Expression is inhibited. | | | | |

According to the present invention, there is provided a method of constructing a nucleus-implanted egg that is a nucleus-implanted egg having 2 (two) haploid genome sets derived from mammal ova and shows excellent production efficiency of adults. Further, according to the present invention, there are also provided a method of constructing a parthenogenetic embryo from the nucleus-implanted egg and a method of producing a parthenogenetic mammal from the parthenogenetic embryo. Mammals obtained by the present invention have a normal reproduction ability.

In particular, according to the present invention, production efficiency of adults with respect to a parthenogenetic embryo is about 7%, and the present invention is technically more significant than the conventional method (W02005/011371) showing a production efficiency of about 1.5%.

### BRIEF DESCRIPTION OF THE DRAWINGS

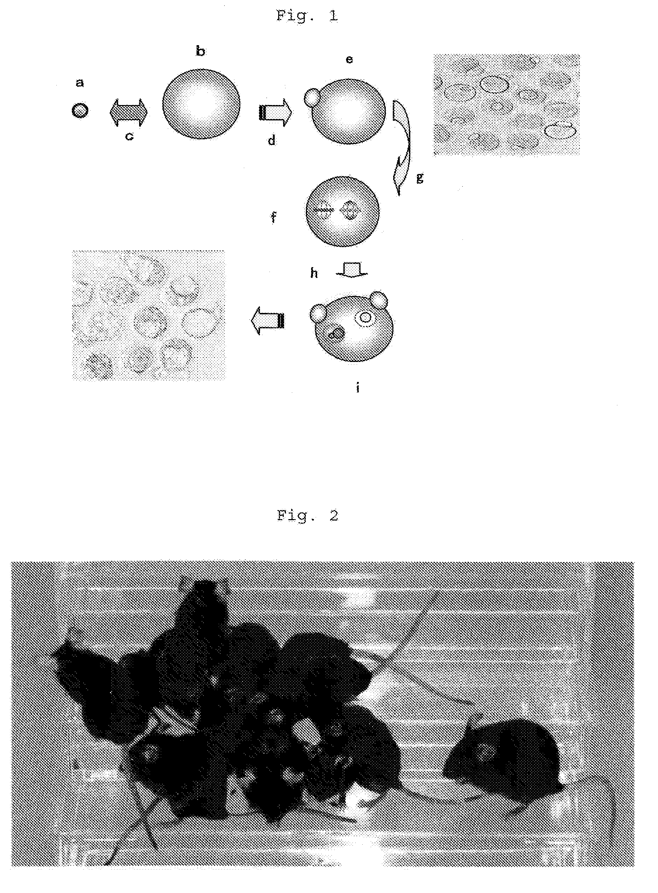

Fig. 1 is a drawing for schematically showing the method of constructing a nucleus-implanted egg, provided by the present invention. In Figs. 1 to 4, symbols a to i represent as follows.
   a: ng ovum derived from a neonate
   b: fg ovum derived from a matured female
   c: Nuclear implanting
   d: Maturing in vitro by culturing
   e: Matured nucleus-substituted ovum
   f: Ovulation ovum
   g: Implanting of MII phase mitotic apparatus
   h: Artificial activation of ovum
   i: Reconstructed ng/fg parthenogenetic embryo a to e correspond to the first step of nuclear implantation in the present invention, and f to i correspond to the second step of nuclear implantation in the present invention.
Fig. 2 is a photograph of a parthenogenetic mouse obtained according to the present invention and an offspring thereof.

### DETAILED DESCRIPTION OF THE PREFERRED EMBODIMENTS

### (First Step of Nuclear Implantation)

This is a step in which a non-grown stage ovum (ng ovum) is introduced into a nucleus-deleted germinal vesicle stage egg (GV stage egg) and matured in vitro by culturing to develop it up to MII phase (second meiosis metaphase).

As a GV stage egg, there can be used an in vivo grown ovum obtained by administering a matured mammal with a pregnant mare ciliary gonadotropic hormone and carrying out super-ovulation treatment or an in vivo grown ovum obtained from the ovary of a matured mammal without any treatment. The above in vivo grown ovum can be collected by incision of an ovarian follicle from a female mammal ovary obtained by super-ovulation treatment or without any treatment with an injection needle using a PBS solution or by suction from an ovarian follicle with an injection needle, or the like. In a GV stage egg having cumulus cells adhering thereto, preferably, the cumulus cells are removed by pipetting with a glass pipette or by oxygen treatment with trypsin-EDTA or the like.

The GV stage egg is subjected to the cutting of zona pellucida and deletion of nucleus to prepare a recipient egg. The zona pellucida can be cut off with a glass knife while observing it through a microscope. The deletion of nucleus can be performed by inserting a nucleus-deleting pipette through a zone pellucida ablation portion and removing it together with a small amount of cytoplasm.

While the ng ovum is preferably an oocyte in a fetal life or an oocyte of a neonate, it can be also collected from the ovary of a matured mammal.

The introduction is preferably carried out by cell fusion. Preferably, the ng ovum is injected into the subzone of the recipient egg together with Sendai virus of Japan (HVJ) and fused.

Then, the ng ovum is matured in vitro by culturing to be developed to MII phase (second meiosis metaphase) . The maturation in vitro by culturing can be carried out in an αMEM culture medium containing 5% of fetal bovine serum (FBS) in a carbon dioxide culturing apparatus. There can be also used an M16 culture medium containing 5% of fetal bovine serum (FBS). In the in vitro maturation by culturing, the egg performs the disintegration of karyotheca, the formation of a mitotic division apparatus, miosis and the releasing of the first polocyte, whereby there can be obtained the first nucleus-implanted egg that has reached MII phase.

The mammal preferably includes non-human animals such as a mouse, a swine, a cow, a sheep, a goat, a rat, a rabbit, and the like.

### (Second Step of Nuclear Implantation)

This step is a step in which an MII phase chromosome is extracted from the first nucleus-implanted egg and introduced into other MII phase egg (fg ovum) to prepare a second nucleus-implanted egg.

The fg ovum is preferably an ovulation ovum from a female matured mammal. The above ovulation ovum can be obtained by administering a matured mammal with a pregnant mare ciliary gonadotropic hormone or human ciliary gonadotropic hormone and carrying out ovulation treatment. On the other hand, as the fg ovum, there can be also used an egg that is developed to MII phase by obtaining an in vivo grown ovum from the ovary of a matured mammal without any treatment and maturing the ovum in a state where it is covered with cumulus cells by in vitro culturing. In the fg ovum, preferably, part of its zona pellucida is cut beforehand.

The introduction can be carried out by the following method. An MII phase chromosome (mitotic apparatus) is sucked from the zona pellucida ablation portion of the first nucleus-implanted egg with a nucleus-deleting pipette. Then, a hemagglutinating virus of Japan is sucked in a tip of a pipette and injected into the MII phase chromosome by inserting the tip through the zone pellucida ablation portion of the fg ovum. These procedures are preferably carried out in a nuclear implanting medium such as an M2 culture medium. Then, culturing is carried out in the M2 culture medium for a predetermined period of time for fusion, so that the second nucleus-implanted egg can be obtained. The second nucleus-implanted egg has a haploid genome set derived from the ng ovum and a haploid genome set derived from the fg ovum.

### (Region A, Region B)

In the present invention, an ng ovum or fg ovum is an ovum in which both a region A and a region B are missing.

The region A is a DNA methylation imprint region that controls expressions of H19 gene and Igf2 gene. The region A is a region having a length of 10 kb and situated between the Igf2 gene and the H19 gene, in the case of a mouse. It is a region from an EcoRI restriction enzyme cleavage site nearest to the 5' side of the H19 gene to an EcoRI restriction enzyme cleavage site in an upstream of about 10 kb.

The H19 gene is described in Leighton P. A. et al, Nature 375, 34-39, 1995. The length of the H19 gene is 3 kb. The Igf2 gene is described in Leighton et al, Nature 375, 34-39, 1995.

The region B is a DNA methylation imprint region that controls expressions of Gtl2 gene and Dlk1 gene. The region B is a region having a length of 4.2 kb and situated between the Dlk1 gene and the Gtl2 gene, in the case of a mouse. It is a region from an Sca1 restriction enzyme cleavage site nearest to the 5' side of the Gtl2 gene to an Sac1 restriction enzyme cleavage site in an upstream of about 4.2 kb.

The Gtl2 gene is described in maternally expressed in Schmidt, J. V. et al, Genes Dev. 14, 1997-2002, 2000. The Dlk1 gene is described in Schmidt, J. V. et al, Genes Dev. 14, 1997-2002, 2000.

The ovum in which both the region A and the region B are missing can be obtained from neonates born by mating the respective gene-deleted mammals with each other. The gene-deleted mammal can be produced by the use of a known target gene recombination method (gene-targeting: e.g., Methods described in Enzymology 225: 803-890, 1993), and for example, such a mouse can be produced as follows.

First, the target sequence of isolated regions A and B is replaced with a neomycin resistance gene (Neor gene), and a thymidine kinase gene (HSV-tk gene) that is a herpes virus is added to the terminal portion of the regions A and B to prepare a targeting vector. The targeting vector is introduced into mouse embryo-stem cells (ES cells), and there are selected cells in which the regions A and B of cellular genome DNA are homologously recombined with the mutant sequence of the targeting vector.

The selection of the above gene-recombined cells can be made by adding G418 to a cell culture medium, removing non-recombined cells having no Neor gene, further adding ganciclovir and removing random-recombined cells in which the HSV-tk gene remains. The regions A and B of the thus-selected gene-recombined cells are a mutant sequence obtained by inserting the Neor gene into the coding sequence thereof and cannot be expressed.

Then, the above gene-recombined ES cells are injected into the initial embryo (blastocyst) of a mouse, and the initial embryo is developed in vivo to an individual to produce a chimera mouse. Then, the chimera mouse and a wild-type mouse are allowed to mate to produce offspring mice, and individual mice having a mutant sequence in the regions A and B are selected from these offspring mice, whereby gene-deleted mice can be obtained.

Although the present invention uses an ovum in which the regions A and B that are expression regulating regions are missing, an ovum in which an H19 gene itself in addition to the regions A and B is missing may be used.

### (Construction of Parthenogenetic Embryo)

The present invention includes a method of constructing a parthenogenetic embryo, which comprises activating the above second nucleus-implanted egg and then developing it in vitro by culturing. Preferably, the ovum is activated with strontium. Specifically, the ovum can be activated by culturing it in an M16 culture medium containing 10 mM of SrCl₂.
Alternatively, the ovum can be activated by electric pulse, ethanol or the like. The development in vitro by culturing can be carried out under conditions of a 5% CO₂, 5% O₂ and 90% N₂ gaseous phase and 37 to 39°C.

### (Production of Parthenogenetic Mammal)

The present invention includes a method of producing a parthenogenetic mammal, which comprises implanting the above parthenogenetic embryo in the uterus of a same kind of mammal, from which the parthenogenetic embryo was constructed, nucleus-implanted egg and Nun tp, and then allowing it to grow. While the mammal for the implantation is not specially limited, it is preferred to use a mammal that is artificially inseminated and then induced to abort with prostaglandin F2α, or the like during the early stage of gestation for synchronization. The mammal preferably includes non-human mammals such as a mouse, a swine, a cow, a sheep, a goat, a rat, a rabbit, and the like.

### EXAMPLES

The present invention will be described with reference to Examples hereinafter. In Examples, mice were used as a mammal.

### Example 1

### (Collection of ng Ova)

Ovaries were collected from one-day-old female neonates obtained by mating mice (Leighton et al., Nature 375: 34-39, 1995) from which 13 Kb (H19 genes and region A) of H19 genes (3 kb) and upstream regions thereof (10 kb) had been deleted according to a target gene recombination method (Methods in Enzymology 225: 803-890, 1993) with mice (Schmidt, J. V. et al, Genes Dev. 14, 1997-2002, 2000) from which a Dlk1-Gtl2 expression regulating region (region B, length: 4.15 Kb, Gtl2 gene upstream regions) had been deleted. The collected ovaries were transferred into a 0.02% EDTA solution and cultured at 37°C for 10 minutes. Then, the ovaries were cut apart with an injection needle and dissociated non-grown stage ova where collected. Ova from which both the region A and the region B had been deleted were selected from the obtained ova and used as ng ova as a donor.

### (Collection of GV Stage Egg)

Matured mice (8 to 12 weeks old, B6D2F1, Charles River/Claire) were administered with pregnant mare ciliary gonadotropic hormone at a dose of 5 to 7.5 IU, and then grown ovarian follicles in the ovaries were cut apart with a 27-gage injection needle to collect GV stage eggs covered each with cumulus cells. The cumulus cells were removed by pipetting, and then the GV stage eggs were cultured in an M2 culture medium (containing 240 µM of dbcAMP and 5% FBS) at 37°C for 2 hours.

### (Nuclear Removal)

A micromanipulator (supplied by Narishige Co., Ltd.) was fixed to an inverted microscope for an operation. First, the zona pellucida each of the GV stage eggs was 15 to 20% cut off with a glass knife. Then, the GV stage eggs were transferred to a nucleus-implanting M2 medium (containing 10 µg/ml of cytochalasin B, 100 ng/ml of colcemid, 240 µM of dbcAMP and 5% FBS) and cultured at 37°C for 15 minutes. By micromanipulation, a nucleus-removing pipette (having a diameter of 25 µm) was inserted through the ablation portion each of the zona pellucida, and the nucleus each of the GV stage eggs was removed together with a small amount of cytoplasm, to prepare recipient eggs.

### (First Step of Nuclear Implantation)

Then, the ng ova were sucked into an implanting pipette (having a diameter of 15 µm), and then hemagglutinating virus of Japan (HVJ: Cosmobio) was sucked into the tip portion of the pipette. The pipette was inserted through the ablation portion of the zona pellucida each of the recipient eggs and pressed to each recipient egg for injection. The thus-obtained nucleus-implanted eggs were transferred to an αMEM culture medium containing 5% FCS and cultured in a carbon dioxide gas incubator at 37°C for 14 hours. The nucleus-implanted eggs developed to the second meiosis metaphase (MII phase) through the steps of disintegration of the nuclear membrane, generation of the mitotic apparatus, miosis and releasing of the first polocyte, to give the first nucleus-implanted eggs.

### (Second Step of Nuclear Implantation)

Matured female mice (8 to 12 weeks old, B6D2F1, Charles River/Claire) were administered with pregnant mare ciliary gonadotropic hormone and human ciliary gonadotropic hormone at a dose of 5 to 7.5 IU each at an interval of 48 hours, and 14 hours after the administration of the human ciliary gonadotropic hormone, oviducts were collected. A mass of ovulation ova covered with cumulus cells were collected from the oviducts, then, the cumulus cells were removed in an M2 culture medium containing 300 µg/ml of hyaluronidase by pipetting, and then, ovulation ova (fg ova) were collected. Part of zona pellucida was cut apart by micromanipulation. The ovulation ova and the first nucleus-implanted eggs were transferred into an implanting M2 culture medium (containing 5 µg/ml of cytochalasin B). A nucleus-deleting pipette (having a diameter of 25 µm) was inserted through the ablation portion of the zona pellucida each of the above first nucleus-implanted eggs, and an MII phase chromosome (mitotic apparatus) was sucked therein. Then, hemagglutinating virus of Japan was sucked into the tip portion of the pipette, and the pipette was inserted into the ablation portion of zone pellucida each of the fg ova to inject the MII phase chromosome. They were transferred to an M2 culture medium and cultured at 37°C for 30 minutes to fuse them, whereby second nucleus-implanted eggs were obtained.
The following Table 4 shows production efficiency of the nucleus-implanted eggs.

**Table 4**

| | First Nucleus-Implanted Eggs | Second Nucleus-Implanted Eggs |
|---|---|---|
| Number of Fused Eggs/Number of Manipulated Eggs (percent) | 322/383 (84.1%) | 238/274 (86.9%) |
| Number of Mature Eggs (percent) | 274 (85.10) | --- |

### Example 2

### (Construction of Parthenogenetic Embryo)

The thus-obtained 238 second nucleus-implanted eggs were cultured in an M16 culture medium containing 10 M of strontium chloride at 37°C for 3 hours to artificially induce activation of the ova. As a result, 221 ova were activated. The ova were in vitro cultured in an M16 culture medium at 37°C for 3 days. As a result, 206 blastocysts were produced.

### Example 3

### (Production of Parthenogenetic Mouse)

The thus-obtained 206 blastocysts were implanted in an uterus of a female mouse at day 2.5 of pseudopregnancy (female mouse that was mated with a vasoligated male according to a conventional method and a day when its copulatory plug was confirmed was a day 0.5), to give birth to 14 normal female neonates, all of which grew to adulthood. Thus, production efficiency was as high as 7%. Analysis of their genes showed that they had deficiencies of expression regulating methylation regions (regions A and B) of H19-IGF2 gene and GTL-DLK1 gene in hetero, so that it was confirmed that the above neonates were neonates from the second nucleus-implanted eggs.

As a result of examining the reproduction abilities of five mice out of these 14 parthenogenetic mice, it was found that all of the tested parthenogenetic mice normally mated, became pregnant and gave birth and it was confirmed that they all had a normal reproduction ability.

According to the present invention, there can be produced an adult parthenogenetic mammal having 2 haploid genome sets derived from ova. Such a mammal is useful as a laboratory animal for analyzing functions of genes. According to the present invention, produced mammals are all female, so that there can be efficiently produced, for example, milking cows that have excellent genes and that are genetically uniform. Further, there can be efficiently produced cows that provide excellent beef cattle. The present invention so promises its use in the livestock industry. When parthenogenetic mammals are produced according to the present invention, non-human mammals are main objects thereof, while it is expected that the present invention will be applied to production of internal organs for implantation in the medical field.

## Claims

1. A method of constructing a nucleus-implanted egg of a mammal, the nucleus-implanted egg having a haploid genome set derived from an ng ovum and a haploid genome set derived from an fg ovum, which comprises the steps of
(1) introducing a non-grown stage egg (ng ovum) into a nucleus-deleted egg in a germinal vesicle stage (GV stage egg) and then developing the obtained egg to an MII phase (second meiosis metaphase) by in vitro culture for development to prepare a first nucleus-implanted egg, and
(2) extracting all of MII phase chromosomes from said first nucleus-implanted egg and introducing it into other MII phase egg (fg ovum) to prepare a second nucleus-implanted egg,
wherein an ovum in which both (A) a DNA methylation imprint region (region A) that controls expressions of H19 gene and Igf2 gene and (B) a DNA methylation imprint region (region B) that controls expressions of Gtl2 gene and Dlk1 gene are deleted is used as the ng ovum or fg ovum.

2. The method of claim 1, wherein the non-grown stage egg (ng ovum) is an oocyte of a neonate.

3. The method of claim 1, wherein the other MII phase egg (fg ovum) is an ovulation ovum.

4. The method of claim 1, wherein the ovum from which both the region A and the region B are deleted is derived from a gene-deleted mammal.

5. A method of constructing a parthenogenetic non-human embryo, which comprises activating the second nucleus-implanted egg obtained by the method recited in claim 1 and then culturing the second nucleus-implanted egg in vitro to develop the second nucleus-implanted egg.

6. A method of producing a parthenogenetic non-human mammal, which comprises implanting the parthenogenetic embryo obtained by the method recited in claim 5 into the uterus of a mammal and growing the parthenogenetic embryo.
